# FASCICULE DE BREVET EUROPEEN

(11) **EP 0 990 429 B1**
(45) Date de publication et mention de la délivrance du brevet: **30.11.2005**
(21) Numéro de dépôt: 99402329.9
(22) Date de dépôt: 23.09.1999
(51) Int. Cl.: A61F 5/441

(54) **Systeme de filtre et d'event de degazage integre dans une poche de recueil pour stomises**
In einem Ostomiebeutel integriertes Filter- und Entlüftungssystem
Filter and gas vent system incorporated in an ostomy bag

(30) Priorité: 30.09.1998 FR 9812214
(43) Date de publication de la demande: 05.04.2000
(73) Titulaire: B. Braun Medical Société Anonyme, 92100 Boulogne Billancourt (FR)
(72) Inventeur: Holtermann, Henri, 64500 Saint-Jean-de-Luz (FR); Hamelin, Claude, 64130 Ascain (FR); Dumartin, Claude, 64500 Saint-Jean-de-Luz (FR)
(74) Mandataire: Goulard, Sophie

(56) Documents cités:
- EP-A- 0 064 044
- EP-A- 0 116 363
- EP-A- 0 130 019
- EP-A- 0 294 257
- US-A- 4 367 742

## Description

La présente invention concerne un système de filtre et d'évent de dégazage adapté à être intégré dans une poche de recueil pour stomisés au cours de sa fabrication.

Les stomisés sont des patients qui ont subi une opération chirurgicale telle qu'une colostomie ou une iléostomie avec création d'un anus artificiel encore appelé par extension " stomie ". Ils sont ensuite équipés d'une poche de recueil constituée par deux parois de matière plastique imperméable qui sont soudées l'une à l'autre le long du pourtour de la poche. L'une de ces parois comporte une ouverture pour le recueil des matières provenant de la stomie. Cette ouverture est entourée d'un protecteur cutané adapté à adhérer de façon pratiquement étanche à la paroi abdominale du stomisé en vue du maintien en place de la poche.

Les matières recueillies dans la poche s'accompagnent de gaz intestinaux dont l'évacuation est rendue nécessaire de façon à éviter un gonflement excessif de la poche. Il convient toutefois de maintenir à l'intérieur de celle-ci un minimum de pression résiduelle. En effet, dans le cas contraire, les deux parois de la poche pourraient se coller l'une à l'autre, ce qui s'opposerait à l'entrée des matières. Un léger effet de coussinet augmente aussi le confort en évitant la transmission des pressions extérieures, notamment celles des vêtements, à la stomie qui demeure sensible. A cette fin, la poche est munie d'un évent de dégazage. Celui-ci est disposé au-dessus de l'ouverture de la poche par laquelle pénètrent les matières de façon que ces dernières tombant par gravité au fond de la poche, elles n'obturent pas l'évent.

Par ailleurs, certains gaz intestinaux dégagent une odeur nauséabonde gênante pour le patient et son entourage. Ainsi munit-on les évents d'un filtre désodorisant placé sur le trajet des gaz de telle sorte que ceux-ci le traversent pour se purifier avant de sortir de la poche. Dans les sytèmes de filtre et d'évent les plus anciens, le filtre était simplement placé sur le trou d'évent pratiqué dans une des parois de la poche, et cela selon les cas à l'extérieur ou l'intérieur de la poche. Puis, à la fois pour réduire le coût de fabrication et pour éviter toute manipulation aux stomisés parfois âgés ou handicapés, on a cherché à les intégrer à la poche au moment même de sa fabrication.

Au nombre de tels systèmes incorporés, on peut citer le Brevet EP 0 068 964 au nom des Laboratoires Biotrol. Ce système se présente sous la forme d'une pastille d'une matière filtrante protégée par un film. Il est pris dans la soudure périphérique de la poche de façon à être à cheval sur cette dernière réalisant ainsi un filtre à passage direct. Sa face de sortie peut déboucher directement à l'extérieur de la poche ou bien à l'intérieur d'une chambre aval faisant partie intégrante de la poche ou la complétant et dont les parois peuvent être perforées pour réaliser un évent.

Ce système a ensuite été amélioré, notamment pour ce qui est de la longueur de parcours des gaz et donc pour ce qui est de sa durée de fonctionnement efficace, par celui objet du Brevet FR 2 615 099. Il se présente sous la forme d'une bande plate et se compose d'une âme filtrante allongée prise entre deux films protecteurs soudés l'un à l'autre de part et d'autre de l'âme. Il est disposé entre les deux parois de la poche de façon à s'étendre horizontalement d'un bord latéral de la poche à l'autre. Il est maintenu dans la soudure périphérique de la poche au niveau de chacun de ces bords. Les extrémités du filtre sont dépourvues de film de sorte que l'âme communique avec l'extérieur de la poche. Les gaz pénètrent dans l'âme par un trou pratiqué dans le film protecteur au centre du filtre. Ainsi parcourent-ils la demi-largeur de la poche avant d'être libérés à l'extérieur par les extrémités du filtre.

Le filtre selon le Brevet FR 2 615 099 qui vient d'être évoqué, souffre cependant d'un inconvénient. La matière qui compose son âme effleure en effet le long du pourtour de la poche. Or, elle contient du charbon actif qui peut se détacher et salir les vêtements. Lors des douches, les particules de charbon actif sont entraînées en plus grandes quantités encore. Il se forme en outre un colmatage des extrémités du filtre qui l'empêche de fonctionner. Son caractère intégré à la poche entraîne alors la nécessité de jeter la poche toute entière. Par ailleurs, les matières liquides à l'intérieur de la poche peuvent cheminer le long du charbon actif et venir suinter à l'extérieur et donc souiller les vêtements tout en provoquant des nuisances olfactives. On pourrait songer à obturer les extrémités du filtre par des opercules amovibles, mais cela n'est pas plus satisfaisant que les filtres rapportés après coup sur la poche, les stomisés étant souvent âgés et infirmes. C'est pourquoi un problème est de développer un système de filtre et d'évent intégré dans lequel l'âme du filtre demeure isolée de l'extérieur de la poche.

Dans ce but, il est proposé selon l'invention un système de filtre et d'évent de dégazage intégré dans une poche de recueil pour stomisés au cours de sa fabrication, ladite poche ayant un bord supérieur essentiellement horizontal ainsi que deux bords latéraux essentiellement verticaux et étant constituée par deux parois parallèles soudées entre elles le long d'un pourtour de la poche, l'une d'elles étant munie d'une ouverture pour l'entrée des matières dans la poche, le filtre ayant la forme d'une bande plate présentant deux extrémités et étant constitué par une âme désodorisante recouverte d'au moins un film protecteur imperméable aux gaz sauf à ses deux extrémités où l'âme est laissée à l'air libre, le filtre étant maintenu horizontal à l'intérieur de la poche dans sa partie située au-dessus de l'ouverture pour l'entrée des matières par deux soudures des deux parois de la poche entre elles pratiquées au niveau des extrémités du filtre, caractérisé en ce que chacune des deux soudures s'étend d'un des bords latéraux de la poche à son bord supérieur et en ce que le filtre est entièrement contenu dans la poche qui protège ainsi ses extrémités.

C'est ainsi que par rapport au système de filtre et d'évent de dégazage connu selon le Brevet FR 2 615 099, le filtre n'est plus maintenu par la soudure périphérique de la poche, mais par les soudures s'étendant des bords latéraux de la poche à son bord supérieur. Le filtre peut dès lors s'arrêter avant la soudure périphérique, ses extrémités débouchant à l'intérieur des compartiments délimités par les soudures de maintien du filtre et la soudure périphérique.

Selon une disposition avantageuse de l'invention, l'une des deux soudures maintenant le filtre est incomplète, un étroit chenal étant laissé à proximité immédiate du bord supérieur pour le passage des gaz. De la sorte, les matières ne peuvent remonter jusqu'à l'extrémité du filtre se trouvant derrière la soudure incomplète et le bord latéral correspondant de la poche. L'âme du filtre se trouve ainsi protégée et ne risque pas de s'encrasser, empêchant la poursuite de l'évacuation des gaz.

Selon une autre disposition avantageuse de l'invention, un trou d'évent est pratiqué dans une des parois de la poche au niveau de celle des extrémités du filtre opposée à l'extrémité maintenue par la soudure incomplète de sorte que les gaz doivent parcourir l'intégralité du filtre pour sortir à l'extérieur de la poche. Il s'ensuit un parcours des gaz plus important que dans le filtre selon le Brevet FR 2 615 099 cité ci-dessus. Au lieu en effet de parcourir la moitié du filtre, les gaz parcourent ici toute sa longueur. Dès lors, à pouvoir de désodorisation égal, le filtre n'a pas besoin d'être aussi long. Cela permet d'économiser sur la matière et donc de réduire le coût global de la production.

Selon encore une autre disposition avantageuse de l'invention, un opercule amovible ferme initialement l'évent de sorte qu'il est possible d'attendre qu'une certaine pression des gaz soit atteinte à l'intérieur de la poche avant d'autoriser l'évacuation des gaz.

De manière également avantageuse, les soudures de maintien du filtre sont rectilignes.

En variante, les soudures de maintien du filtre sont incurvées.

Enfin, avantageusement, l'âme du filtre est prise entre deux films protecteurs soudés entre eux de part et d'autre de l'âme.

D'autres avantages et caractéristiques de la présente invention apparaîtront à la lecture de la description non limitative suivante de formes de réalisation de systèmes de filtre et d'évent de dégazage intégré selon l'invention, description faite en référence aux dessins annexés dans lesquels:
- la figure 1 est une vue côté abdomen de l'utilisateur d'une poche de recueil ouverte comprenant un système de filtre et d'évent de dégazage intégré selon l'invention,
- la figure 2 est une vue côté vêtements de la poche de la figure 1,
- la figure 3 est une vue de côté de la poche de la figure 1,
- la figure 4 est une vue côté vêtements d'une poche de recueil fermée comprenant un système de filtre et d'évent de dégazage intégré selon l'invention,
- la figure 5 est une vue de détail d'un système de filtre et d'évent de dégazage selon l'invention, et
- la figure 6 est une vue en coupe du filtre de la figure 5.

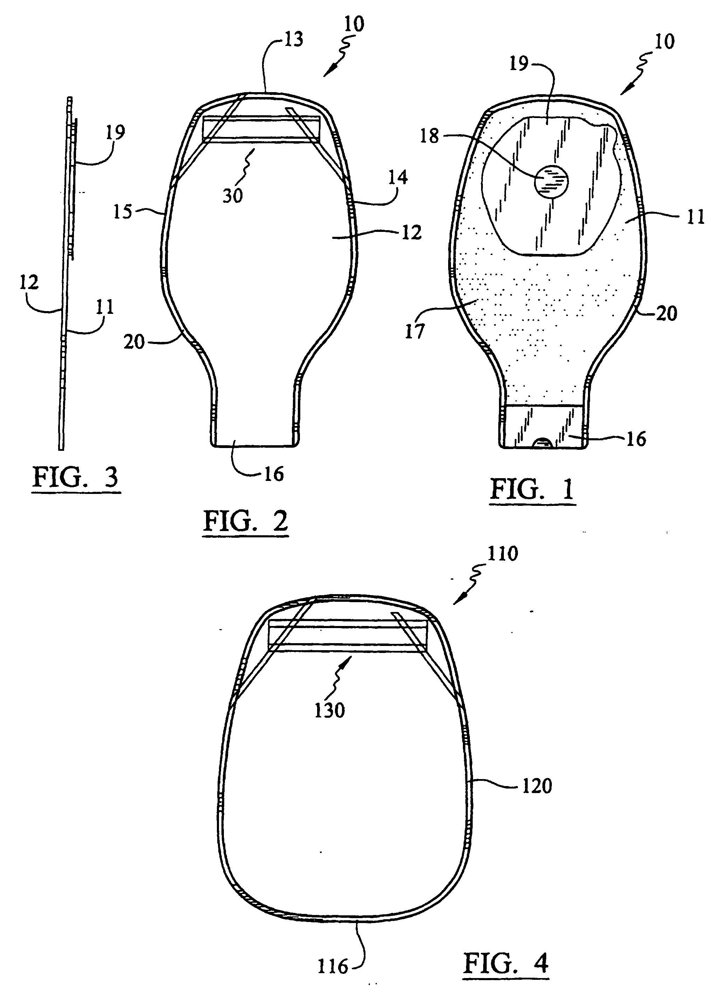

La figure 1 montre une poche de recueil 10 vue du côté destiné à venir au contact de l'abdomen de l'utilisateur. Comme cela est également visible sur la vue de côté de la figure 3, la poche est constituée de deux parois parallèles 11 et 12 soudées l'une à l'autre le long de son pourtour 20. Le bord inférieur 16 de la poche n'est cependant pas fermé et la poche est dite pour cette raison " ouverte ". Ce bord se prolonge en effet en une extension dont les côtés sont parallèles pour permettre la vidange de la poche. L'extension est maintenue repliée sur elle-même par un clamp (non représenté) afin de refermer la poche lorsque celle-ci est en place pour le recueil des matières. Les parois 11 et 12 sont réalisées dans une matière plastique étanche aux liquides et aux gaz. Afin d'éviter le contact désagréable de cette matière avec la peau de l'abdomen de l'utilisateur, la paroi 11 correspondante est recouverte d'un revêtement 17 en tissu, en feutre ou en non-tissé.

Toujours du côté abdomen, la paroi 11 présente une ouverture 18 pour l'entrée des matières dans la poche. Un protecteur cutané 19 entoure l'ouverture 18. Comme visible sur la figure 3, il saille sur la paroi 11 à laquelle il est assujetti notamment par soudure ou collage. Le protecteur 19 peut être constitué simplement d'un substrat recouvert d'un adhésif sensible à la pression ou comporter en outre un absorbant, constitué par exemple par des hydrocolloïdes. Il permet de maintenir la poche sur l'abdomen de l'utilisateur, la stomie venant en regard de l'ouverture 18, et cela de façon relativement étanche.

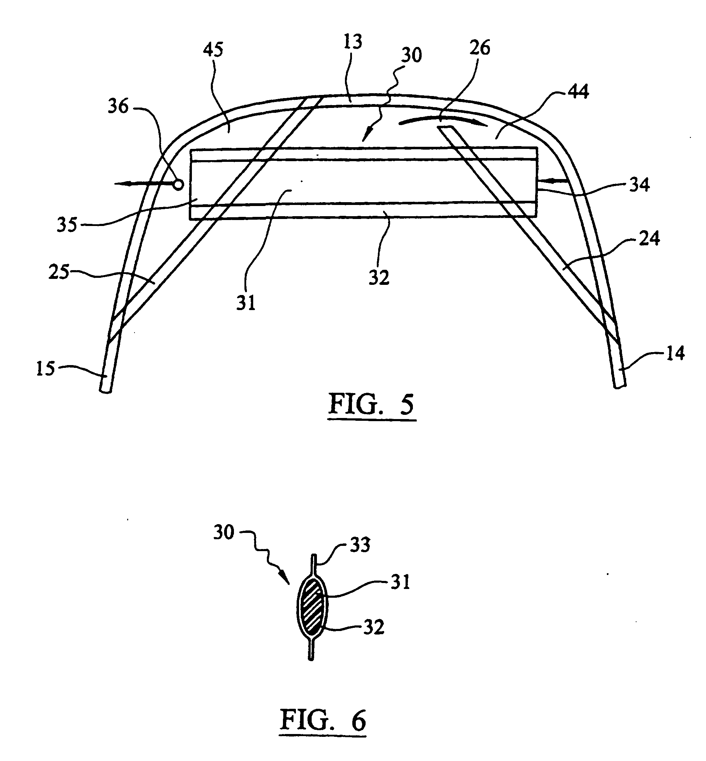

La figure 2 montre la même poche, mais vue cette fois du côté des vêtements de l'utilisateur. Le système de filtre et d'évent de dégazage selon l'invention y est visible en partie haute de la poche. Ce dernier est représenté plus en détail sur la figure 5. Il peut être adapté de la même façon à une poche dite " fermée " telle que celle de la figure 4, c'est-à-dire dont le bord inférieur 116 est soudé comme le reste du pourtour 120 de la poche.

Ce système comporte tout d'abord un filtre 30 en forme de bande plate. Il est constitué comme le filtre objet du Brevet FR 2 615 099 dont il a déjà été question dans le préambule de ce texte. En résumé et comme le montre mieux la coupe de la figure 6, il se compose d'une âme 31 prise entre deux films protecteurs 32 soudés entre eux de part et d'autre de l'âme au niveau de lisières 33. L'âme présente des qualités de résistance aux efforts de même qu'aux agents chimiques. C'est par excellence une matière plastique expansée à alvéoles ouvertes. Ces dernières sont avantageusement remplies de charbon actif associé le cas échéant à de l'oxyde ferrique en vue de conférer au filtre des qualités de désodorisation. Le film est quant à lui plutôt une matière plastique étanche au gaz. Aux extrémités du filtre, soit le film qui recouvre l'âme est poreux, soit l'âme est laissée libre exactement comme dans le Brevet FR 2 615 099.

Le filtre est par ailleurs maintenu horizontalement entre les deux parois 11 et 12 de la poche grâce à deux soudures 24 et 25 de ces parois entre elles. Chacune de ces soudures s'étendent depuis un bord latéral 14 ou 15 de la poche jusqu'au bord supérieur 13 essentiellement horizontal. Sur la figure 5, les soudures 24 et 25 sont rectilignes, mais rien ne s'oppose à ce qu'elles soient incurvées, par exemple avec une concavité orientée vers l'extérieur de la poche. Ainsi chacune des extrémités 34 et 35 du filtre se retrouve-t-elle dans un compartiment 44 ou 45 délimité par la soudure et le bord latéral correspondants. En d'autres termes, les extrémités demeurent à l'intérieur de la poche qui isole ainsi le charbon actif des vêtements ou de l'eau des douches.

Enfin, de façon caractéristique du système selon la présente invention, une des soudures, la soudure 24 située à droite sur la figure 5, n'est pas complète. Un étroit chenal 26 est laissé libre entre la soudure 24 et celle du bord supérieur 13. Ce chenal autorise le passage des gaz à évacuer depuis l'intérieur de la poche jusque dans le compartiment 44 contenant l'extrémité 34 du filtre. Le parcours des gaz est représenté par des flèches sur la figure 5. Une fois le chenal passé, ils pénètrent dans le filtre par son extrémité 34 pour en ressortir à l'autre extrémité 35 du filtre. Cette dernière est située dans le compartiment 45 au niveau duquel la paroi 12 côté vêtements de la poche comporte un trou d'évent 36. Au demeurant, le trou d'évent 36 peut n'être percé qu'une fois atteint un certain niveau de pression à l'intérieur de la poche. Il peut également être initialement obturé par un opercule amovible que l'utilisateur retire au moment voulu.

Selon une variante de l'invention non représentée, un seul film protecteur 32 est prévu qui est directement soudé à la paroi 12 côté vêtements de la poche. Il demeure cependant plus économique de fabriquer au kilomètre une bande filtrante présentant la coupe de la figure 6 et de la débiter à la longueur des filtres.

Il est clair que l'invention n'est nullement limitée aux formes de réalisation décrites ci-dessus en référence aux dessins annexés, mais qu'elle englobe toutes les modifications et variantes issues du même principe de base.

## Revendications

1. Système de filtre et d'évent de dégazage intégré dans une poche de recueil pour stomisés au cours de sa fabrication,
- ladite poche (10) ayant un bord supérieur (13) essentiellement horizontal ainsi que deux bords latéraux (14,15) essentiellement verticaux et étant constitués par deux parois (11,12) parallèles soudées entre elles le long d'un pourtour (20) de la poche (10), l'une d'elles étant munie d'une ouverture (18) pour l'entrée des matières dans la poche (10),
- le filtre (30) ayant la forme d'une bande plate présentant deux extrémités (34,35) et étant constitué par une âme (31) désodorisante recouverte d'au moins un film protecteur (32) imperméable aux gaz sauf à ses deux extrémités (34,35) où l'âme est laissée à l'air libre,
- le filtre (30) étant maintenu horizontal à l'intérieur de la poche (10) dans sa partie située au-dessus de l'ouverture (18) pour l'entrée des matières par deux soudures (24,25) des deux parois (11,12) de la poche (10) entre elles pratiquées au niveau des extrémités (34,35) du filtre (30),
**caractérisé en ce que**
- chacune des deux soudures (24,25) s'étend d'un des bords latéraux (14,15) de la poche (10) à son bord supérieur (13), et **en ce que**
- le filtre (30) est entièrement contenu dans la poche (10) qui protège ainsi ses extrémités (34,35).

2. Système de filtre et d'évent de dégazage intégré selon la revendication 1, **caractérisé en ce que** l'une des deux soudures (24) maintenant le filtre (30) est incomplète, un étroit chenal (26) étant laissé à proximité immédiate du bord supérieur (13) pour le passage des gaz.

3. Système de filtre et d'évent de dégazage intégré selon la revendication 2, **caractérisé en ce qu'**un trou (36) d'évent est pratiqué dans une des parois (11,12) de la poche (10) au niveau de celle des extrémités (35) du filtre (30) opposée à l'extrémité (34) maintenue par la soudure incomplète (24) de sorte que les gaz doivent parcourir l'intégralité du filtre pour sortir à l'extérieur de la poche (20).

4. Système de filtre et d'évent de dégazage intégré selon la revendication 3, **caractérisé en ce qu'**un opercule amovible ferme initialement le trou (36) d'évent de sorte qu'il est possible d'attendre qu'une certaine pression des gaz soit atteinte à l'intérieur de la poche avant d'autoriser l'évacuation des gaz.

5. Système de filtre et d'évent de dégazage intégré selon l'une des revendications 1 à 4, **caractérisé en ce que** les soudures de maintien du filtre sont rectilignes.

6. Système de filtre et d'évent de dégazage intégré selon l'une des revendications 1 à 4, **caractérisé en ce que** les soudures de maintien du filtre sont incurvées.

7. Système de filtre et d'évent de dégazage intégré selon l'une des revendications précédentes, **caractérisé en ce que** l'âme du filtre est prise entre deux films protecteurs soudés entre eux de part et d'autre de l'âme.

## Patentansprüche

1. Während der Herstellung in einem Ostomiebeutel integriertes Filter- und Entlüftungssystem,
- bei dem der Beutel (10) einen im Wesentlichen horizontalen oberen Rand (13) sowie zwei im Wesentlichen vertikale seitliche Ränder (14, 15) aufweist und durch zwei entlang einer Umfangslinie (20) des Beutels (10) miteinander verschweißte Wände (11, 12) gebildet ist, wobei die eine mit einer Öffnung (18) für den Eintritt von Material in den Beutel (10) ausgestattet ist,
- bei dem das Filter (30) die Gestalt eines zwei Enden (34, 35) aufweisenden flachen Bandes hat und durch eine desodorierende Innenlage (31) gebildet ist, die bis auf die beiden Enden (34, 35), an denen die Innenlage freiliegt, mit einem gasundurchlässigen Schutzfilm (32) bedeckt ist,
- bei dem das Filter (30) im Innern des Beutels (10) mit seinem oberhalb der Öffnung (18) horizontal gehalten ist, die zum Eintritt des Materials durch zwei Fugen (24, 25) in den beiden Wänden (11, 12) des Beutels (10) eingerichtet ist, die zwischen ihnen im Bereich der Enden (34, 35) des Filters (30) eingebracht sind,
**dadurch gekennzeichnet, dass**
- sich jede der beiden Fugen (24, 25) von einem der seitlichen Ränder (14, 15) des Beutels (10) bis zu dessen oberen Rand (13) erstreckt und dass
- das Filter (30) vollständig von dem Beutel (10) aufgenommen ist, der somit die Enden (34, 35) schützt.

2. Integriertes Filter- und Enflüftungssystem nach Anspruch 1, **dadurch gekennzeichnet, dass** eine der beiden das Filter (30) haltenden Fugen (24) unvollständig ist, wobei zum Durchlass von Gas im unmittelbaren Bereich um den oberen Rand (13) ein gerader Durchlass (26) belassen ist.

3. Integriertes Filter- und Entlüftungssystem nach Anspruch 2, **dadurch gekennzeichnet, dass** in einem der Wände (11, 12) des Beutels (10) im Bereich des Endes (35) des Filters (30), das dem durch die unvollständige Fuge (24) gehaltenen Ende (34) gegenüber liegt, ein Entlüflungsloch (36) eingebracht ist, so dass die Gase das gesamte Filter durchlaufen müssen, um aus dem Beutel (20) nach außen zu gelangen.

4. Integriertes Filter- und Entlüftungssystem nach Anspruch 3, **dadurch gekennzeichnet, dass** ein feststehender Verschluss das Entlüftungstoch (36) anfänglich verschließt, so dass es möglich ist, zu warten, bis im Inneren des Beutels ein gewisser Gasdruck erreicht ist, bevor ein Auslass der Gase erfolgt.

5. Integriertes Filter- und Entlüflungssystem nach einem der Ansprüche 1 bis 4, **dadurch gekennzeichnet, dass** die das Filter haltenden Fugen geradlinig sind.

6. Integriertes Filter- und Entlüflungssystem nach einem der Ansprüche 1 bis 4, **dadurch gekennzeichnet, dass** die das Filter haltenden Fugen gebogen sind.

7. Integriertes Filter- und Entlüflungssystem nach einem der vorangehenden Ansprüche, **dadurch gekennzeichnet, dass** die Filterinnenlage zwischen zwei Schutzfilmen angeordnet ist, die beidseitig der Innenlage miteinander verschweißt sind.

## Claims

1. Filtering and degassing vent system integrated during its manufacture into a receiving bag for ostomates,
- the said bag (10) having a substantially horizontal upper edge (13) as well as two substantially vertical lateral edges (14, 15) and consisting of two parallel walls (11, 12) welded together along a periphery (20) of the bag (10), one of these being provided with an opening (18) for materials to enter the bag (10),
- the filter (30) having the form of a flat strip having two ends (34, 35) and consisting of a de-odorising core (31) covered with at least one protective film (32) impervious to gases except at its two ends (34, 35) where the core is left open to the air,
- the filter (30) is held horizontal inside the bag (10) in its part situated above the opening (18) for the entry of materials by two welds (24, 25) holding the two walls (11, 12) of the bag (10) together, made in the region of the ends (34, 35) of the filter (30),
**characterized in that**
- each of the two welds (24, 25) extends from one of the lateral edges (14, 15) of the bag (10) to its upper edge (13) and **in that**
- the filter (30) is entirely contained within the bag (10) which therefore protects its ends (34, 35).

2. Integral filtering and degassing vent system according to claim 1, **characterized in that** one of the two welds (24) holding the filter (30) is incomplete, a narrow channel (26) being left in the immediate vicinity of the upper edge (13) for the passage of gases.

3. Integral filtering and degassing vent system according to claim 2, **characterized in that** a vent hole (36) is made in one of the walls (11, 12) of the bag (10) in the region of the particular end (35) of the filter (30) opposite the end (34) held by the incomplete weld (24) so that the gases must pass through all the filter in order to leave the bag (20) to the outside.

4. Integral filtering and degassing vent system according to claim 3, **characterized in that** a detachable seal initially closes the vent hole (36) so that it is possible to wait for a certain pressure of the gases to be reached inside the bag before evacuation of the gases is permitted.

5. Integral filtering and degassing vent system according to one of claim 1 to 4, **characterized in that** the welds holding the filter are rectilinear.

6. Integral filtering and degassing vent system according to one of claim 1 to 4, **characterized in that** the welds holding the filter are curved inwards.

7. Integral filtering and degassing vent system according to one of the preceding claims, **characterized in that** the core of the filter is held between two protective films welded together either side of the core.
